# EUROPÄISCHE PATENTSCHRIFT

(11) **EP 1 903 115 B1**
(45) Veröffentlichungstag und Bekanntmachung des Hinweises auf die Patenterteilung: **09.03.2011**
(21) Anmeldenummer: 06121093.6
(22) Anmeldetag: 22.09.2006
(51) Int. Cl.: C12P 21/00, C07K 16/00

(54) **Verfahren zur fermentativen Herstellung von Antikörpern**
Process for the fermentative production of antibodies
Procédé pour la production des anticorps par fermentation

(43) Veröffentlichungstag der Anmeldung: 26.03.2008
(73) Patentinhaber: Wacker Chemie AG, 81737 München (DE)
(72) Erfinder: Daßler Dr. Tobias, 81825 München (DE); Wich Dr., Günter, 81377 München (DE)
(74) Vertreter: Potten, Holger

(56) Entgegenhaltungen:
- EP-A1- 0 497 757
- WO-A-2005/038031
- US-A- 5 693 493
- US-A1- 2003 073 164
- SIMMONS L C ET AL: "Expression of full-length immunoglobulins in Escherichia coli: rapid and efficient production of aglycosylated antibodies" JOURNAL OF IMMUNOLOGICAL METHODS, ELSEVIER, AMSTERDAM, NL, Bd. 263, Nr. 1-2, 1. Mai 2002 (2002-05-01), Seiten 133-147, XP004354391 ISSN: 0022-1759
- SHOKRI A ET AL: "Cell and process design for targeting of recombinant protein into the culture medium of Escherichia coli." APPLIED MICROBIOLOGY AND BIOTECHNOLOGY, Bd. 60, Nr. 6, Februar 2003 (2003-02), Seiten 654-664, XP009078758 ISSN: 0175-7598

## Beschreibung

Die Erfindung betrifft ein Verfahren zur fermentativen Herstellung von Volllängen-Antikörpern unter Verwendung von E*scherichia coli* Stämmen, die zur Abgabe von Proteinen ins Fermentationsmedium befähigt sind.

Der Markt für rekombinante Proteinpharmazeutika (Biologics) ist in den letzten Jahren stark gewachsen. Aufgrund der immer noch sehr hohen Produktionskosten für Pharmawirkstoffe auf Proteinbasis wird jedoch laufend nach effizienteren und damit kostengünstigeren Verfahren und Systemen für deren Herstellung gesucht.

Eine besonders wichtige Klasse von Proteinen sind Antikörper. Antikörper werden in der Forschung, in der Diagnostik und als Therapeutikum in großem Umfang eingesetzt, so dass besonders effiziente und in technischem Ausmaß mögliche Produktionsverfahren notwendig sind.

Bei Antikörpern wird zwischen Volllängen-Antikörpern und Antikörperfragmenten unterschieden. Volllängen-Antikörper bestehen aus vier Proteinketten, zwei identischen schweren Ketten und zwei identischen leichten Ketten. Die verschiedenen Ketten sind durch Disulfidbrücken miteinander verbunden. Jede schwere Kette ist aus einer variablen Region (V_{H}) und einer konstanten Region, welche die drei Domänen CH1, CH2 und CH3 umfasst, aufgebaut. Die Region der schweren Kette, die die Domänen CH2 und CH3 umfasst und die auch als Fc-Region bezeichnet wird, ist nicht an der Antigen-Bindung beteiligt, sondern hat andere Funktionen, wie z. B. die Aktivierung des Komplement-Systems. Jede leichte Kette ist aus einer variablen Region (V_{L}) und einer konstanten Region, die die Domäne C_{L} umfasst, aufgebaut. Abhängig von der Aminosäure-Sequenz der schweren Kette werden Antikörper (Immunglobuline) in fünf Klassen eingeordnet: IgA, IgD, IgE, IgG und IgM. Unter dem Begriff Volllängen-Antikörper sind alle Antikörper zu verstehen, bei denen die leichten Ketten jeweils die Domänen V_{L} und C_{L} umfassen und die schweren Ketten substantiell aus den Domänen V_{H}-CH1-CH2-CH3 aufgebaut sind und der Antikörper somit neben der Eigenschaft ein spezifisches Antigen binden zu können, auch in der Lage ist, andere Funktionen (z. B. die Aktivierung des Komplement-Systems) auszuführen.

Dem gegenüber bestehen Antikörperfragmente aus lediglich einem Teil eines Volllängen-Antikörpers, üblicherweise aus dem Teil, der die Antigen-Bindestelle mit umfasst.

Aufgrund seiner sehr gut untersuchten Genetik und Physiologie, der kurzen Generationszeit und der einfachen Handhabung, ist das Gram-negative Enterobakterium *Escherichia coli* gegenwärtig der am häufigsten verwendete Organismus zur Produktion rekombinanter Proteine. Dieser Organismus wird ebenfalls zur Produktion von Antikörper-Fragmenten benutzt.

Im Gegensatz zu Antikörper-Fragmenten gibt es bislang nur sehr wenige Versuche, Volllängen-Antikörper in E. coli zu produzieren. Aufgrund der Größe und komplexen Struktur von Volllängen-Antikörpern ist es schwierig, zu korrekt gefalteten und assemblierten Antikörpern zu kommen. Eine cytoplasmatische Produktion in E. coli ist dabei nicht möglich, da E. coli im Cytoplasma keine Disulfid-Brücken ausbildet. WO02/061090 beschreibt die periplasmatische Produktion von Volllängen-Antikörpern in E. coli. Dazu wird ein speziell gestalteter Expressionsvektor verwendet, auf dem die leichte Kette und die schwere Kette unabhängig voneinander in unterschiedlichen Promoter-Cistron Paaren exprimiert werden. Die beiden Ketten sind dabei jeweils an Signalpeptide fusioniert und werden über den üblichen Sec-Pathway in E. coli ins Periplasma transportiert, wo die Faltung und Assemblierung stattfindet. Zur Gewinnung der Antikörper ist es dabei nötig, die Zellen aufzuschließen. Die Ausbeuten lagen bei maximal 156 mg/l. Höhere Ausbeuten bis 880 mg/l wurden nur erreicht, wenn zusätzlich zu den Antikörper-Ketten periplasmatische Faltungshelfer (Chaperone), wie die dsb-Proteine oder FkpA, auf Plasmiden koexprimiert wurden. Der Antikörper muss aus der Vielzahl der anderen E. coli Proteine aufgereinigt werden.

Dieses Verfahren weist für die technische Produktion von Antikörpern den Nachteil auf, dass die E. coli-Zellen aufgeschlossen werden müssen und das Zielprotein aus der Vielzahl anderer Proteine von E. coli gereinigt werden muss. Eine eventuelle Koexpression von periplasmatischen Chaperonen erschwert diese Aufreinigung durch zusätzliche erhebliche Mengen an unerwünschten Proteinen noch weiter.

Aufgabe der vorliegenden Erfindung war es, ein Verfahren zur Verfügung zu stellen, welches die Herstellung eines korrekt gefalteten und assemblierten Volllängen-Antikörpers ermöglicht und nicht mit den Nachteilen des Stands der Technik behaftet ist.

Diese Aufgabe wird gelöst durch ein Verfahren, welches dadurch gekennzeichnet ist, dass ein *E. coli*-Stamm, welcher ausgewählt ist aus der Gruppe BLR, K802, WCM100, MM28, RV308, RR1, "Leaky"-Mutante von E. coli-Stämmen und welcher periplasmatische Proteine ins Medium abgibt, enthaltend ein Gen kodierend für die schwere Kette eines Antikörpers funktionell verknüpft mit einer Signalsequenz kodierend für ein Signalpeptid sowie ein zweites Gen kodierend für die leichte Kette eines Antikörpers, funktionell verknüpft mit einer Signalsequenz kodierend für ein Signalpeptid, in einem Kulturmedium fermentiert wird, wobei der *E. coli*-Stamm einen Volllängen-Antikörper in das Kulturmedium ausscheidet und der Volllängen Antikörper vom Kulturmedium abgetrennt wird.

Unter einem E. coli-Stamm, der periplasmatische Proteine ins Medium abgibt, ist im Sinne der vorliegenden Erfindung ein E. coli-Stamm zu verstehen, der nach einer Fermentation eine höhere Konzentration von periplasmatischen Proteinen im Nährmedium aufweist, als der Stamm E. coli W3110 (ATCC 27325).

Es handelt sich um einen der folgenden E. coli-Stämme:
- BLR: Novagen
- K802: CGSC* # 5610
- WCM100: herstellbar gemäß EP0338410B1
- MM28: CGSC* # 5892
- RV308: ATCC** # 31608; EP0677109B1
- RR1: ATCC** # 31434
- "Leaky"-Mutanten von E. coli-Stämmen
   * käuflich erhältlich über das E. coli Genetic Stock Center CGSC (830 Kline Biology Tower, MCD Biology Department, 266 Whitney Ave., PO box 208103, Yale University, New Haven),
   ** käuflich erhältlich über LGC Promochem, Mercatorstr. 51, 46485 Wesel, Deutschland.

Unter dem Begriff "Leaky"-Mutanten werden E. coli-Mutanten zusammengefasst, die aufgrund von Mutationen in Stukturelementen der äußeren Zellmembran oder der Zellwand vermehrt periplasmatische Proteine ins Medium abgeben (Shokri et al., Appl. Microbiol. Biotechnol., 2003, 60, 654 - 664). Bevorzugte Vertreter sind E. coli Stämme mit einer Mutation in einem der folgenden Gene : omp-Gene, tol-Gene, excD-Gen, excC-Gen, lpp-Gen, env-Gene und lky-Gene.

Besonders bevorzugt handelt es sich um einen der folgenden E. coli-Stämme:
- JF733 = CGSC* #6047
- A592 = CGSC* # 4923
- A593 = CGSC* # 4924
- A586 = CGSC* # 4925
- CAG12184 = CGSC* # 7437
- G11e1 = CGSC* # 5169
- JE5511 = CGSC* # 6673
- E610 = CGSC* # 6669
- E623 = CGSC* # 6671
- JE5505 = CGSC* # 6672
- PM61 = CGSC* # 6628
- PM61R = CGSC* # 6629
- JP1228 = CGSC* # 6703
- 207 = CGSC* # 6686
- AE84064 = CGSC* # 6575

Dass E.coli-Stämme, die periplasmatische Proteine ins Medium abgeben, für die Produktion von Volllängen-Antikörpern benutzt werden können, ist aus folgendem Grund völlig überraschend. Die Sekretion eines rekombinanten Proteins in das Periplasma von E. coli erfolgt üblicherweise durch den Sec-Apparat der Wirtszelle. Dabei wird das Gen des gewünschten Proteins mit einer Signalsequenz von solchen Proteinen funktionell verknüpft, die normalerweise von *E*. *coli* mit Hilfe des Sec-Apparates exportiert werden. Nach erfolgter Sekretion in das Periplasma wird von einer Signalpeptidase (z. B. LepB bei E. coli) das jeweilige Signalpeptid abgespalten. Die Proteine werden so im ungefalteten Zustand durch die Cytoplasma-Membran transportiert. Im Periplasma werden diese Proteine dann unter Zuhilfenahme periplasmatischer Chaperone, wie zum Beispiel Disulfid-Isomerasen (Dsb-Proteine) oder Peptidylprolylcis,trans-Isomerasen (z. B. FkpA) in die richtige Sekundär-, Tertiär- und Quartärstruktur gefaltet und assembliert. Dieser Vorgang ist bei der Produktion von Volllängen-Antikörpern besonders aufwendig, da bei solchen Proteinen die einzelnen Proteinketten mittels des Sec-Systems zunächst i) unabhängig voneinander in das Periplasma transportiert, ii) dort richtig gefaltet werden müssen, iii) die intrapeptidischen Disulfidbrücken richtig ausgebildet, iv) zwei der leichten Ketten mit zwei der schweren Ketten in der korrekten Form assembliert und v) die interpeptidischen Disulfidbrücken korrekt ausgebildet werden müssen. Der Fachmann ist daher bisher davon ausgegangen, dass die Freisetzung der Proteine in das Medium mit solchen komplexen Faltungs- und Assemblierungsvorgängen interferiert und somit die Abgabe korrekt gefalteter Antikörper in größerer Ausbeute ins Fermentationsmedium nicht möglich ist. Dies um so mehr, als der Stand der Technik lehrt, dass selbst bei der Produktion von Volllängen-Antikörpern im Periplasma hohe Ausbeuten an korrekt gefalteten Antikörpern nur erhalten werden, wenn zusätzlich periplasmatische Chaperone koexprimiert werden (WO 02/061090).

Überraschend wurde nun gefunden, dass bei Herstellung von Volllängen-Antikörpern mittels des erfindungsgemäßen Verfahrens extrazelluläre, korrekt gefaltete, assemblierte und funktionelle Antikörper in Ausbeuten von > 160 mg/l bereits ohne Koexpression von periplasmatischen Chaperonen erhalten werden. Die Ausbeuten können durch Koexpression von periplasmatischen Chaperonen aber weiter gesteigert werden. Bevorzugte Volllängen-Antikörper sind Antikörper der IgG- und IgM-Klasse, inbesonders bevorzugte der IgG-Klasse.

Für die Sekretion der leichten und der schweren Kette von Antikörpern aus dem Cytoplasma in das Periplasma ist es notwendig, das 5'-Ende des jeweiligen Gens der zu produzierenden Kette *in frame* mit dem 3'-Ende einer Signalsequenz für den Protein-Export zu verknüpfen. Dazu sind prinzipiell die Gene aller Signalsequenzen geeignet, die in *E. coli* eine Translokation des Zielproteins mit Hilfe des *sec*-Apparats ermöglichen. Verschiedene Signalsequenzen sind im Stand der Technik beschrieben, so z. B. die Signalsequenzen der folgenden Gene: *phoA, ompA, pelB, ompF, ompT, lamB, malE,* Staphylococcal protein A, StII und andere (Choi & Lee, 2004). Bevorzugt sind die Signalsequenzen des *phoA-* und *ompA*-Gens von *E. coli,* besonders bevorzugt ist die Signalsequenz für eine Cyclodextrin-Glycosyltransferase (CGTase) aus *Klebsiella pneumoniae* M5a1 mit der Sequenz SEQ ID NO: 1 (EP 0448093). Dabei können die Gene der leichten und schweren Kette am 5'-Ende mit unterschiedlichen Signalsequenzen verknüpft sein oder dergleichen, bevorzugt ist die Verknüpfung mit unterschiedlichen Signalsequenzen, besonders bevorzugt ist die Verknüpfung der einen Kette mit der Signalsequenz des *phoA-* oder ompA-Gens von *E. coli* und die Verknüpfung der anderen Kette mit der Signalsequenz für eine Cyclodextrin-Glycosyltransferase (CGTase) aus *Klebsiella pneumoniae* M5a1 mit der Sequenz SEQ ID NO: 1.

Die Herstellung der DNS-Moleküle, die eine Fusion aus einer Signalsequenz und dem jeweiligen Gen der rekombinanten Antikörperpeptidkette umfasst, erfolgt nach dem Fachmann bekannten Methoden. So kann das jeweilige Gen der rekombinanten schweren Antikörper-Peptidkette und das jeweilige Gen der leichten Antikörper-Peptidkette zunächst mittels PCR unter Verwendung geeigneter Oligonukleotide als Primer amplifiziert und anschließend mit gängigen molekularbiologischen Techniken jeweils mit einem DNA-Molekül, das die Sequenz eines Signalpeptids umfasst und das auf analoge Weise wie das Gen der Antikörper-Peptidkette erzeugt wurde, derart verknüpft werden, dass eine *in frame-*Fusion, d.h. ein durchgehender Leserahmen umfassend die Signalsequenz und die Sequenz der jeweilige Peptidkette, entsteht. Diese Signalsequenz-Peptidketten-Genfusionen können dann entweder in einen Vektor, z. B. ein Plasmid, eingebracht werden, oder direkt nach bekannten Methoden in das Chromosom der Wirtszelle integriert werden. Dabei können das Fusionsgen, das die schwere Kette beinhaltet, und das Fusionsgen, das die leichte Kette beinhaltet, auf zwei getrennten, aber miteinander kompatiblen Plasmiden kloniert werden oder sie können auf einem Plasmid kloniert werden. Werden beide Genfusionen in ein Plasmid eingebracht, können sie in einem Operon zusammengefasst werden oder sie können in jeweils separaten Cistronen exprimiert werden. Bevorzugt ist hier ein Zusammenfassen in einem Operon, besonders bevorzugt ist ein Operon, das proximal zur Transkriptionsstartstelle das Fusionsgen enthaltend die schwere Kette des jeweiligen Antikörpers und distal das Fusionsgen enthaltend die leichte Kette des jeweiligen Antikörpers hat. Genauso können die beiden Genkonstrukte in einem Operon zusammengefasst oder in jeweils separaten Cistronen ins Chromosom der Wirtszelle integriert werden. Bevorzugt ist auch hier ein Zusammenfassen in einem Operon, besonders bevorzugt ist ein Operon, das proximal zur Transkriptionsstartstelle das Fusionsgen enthaltend die schwere Kette des jeweiligen Antikörpers und distal das Fusionsgen enthaltend die leichte Kette des jeweiligen Antikörpers hat.

Vorzugsweise wird das DNS Expressionskonstrukt bestehend aus einer Signalsequenz und einem Antikörper-Peptidketten-Gen mit in *E. coli* funktionellen Expressionssignalen versehen (Promotor, Transkriptions-, Translationsstart, Ribosomenbindestelle, Terminator) .

Als Promotoren eignen sich alle dem Fachmann bekannten Promotoren, wie einerseits beispielsweise induzierbare Promotoren wie der lac-, tac-, trc-, lambda PL, ara- oder tet-Promotor oder davon abgeleitete Sequenzen. Andererseits kann auch eine dauerhafte Expression erfolgen durch Verwendung eines konstitutiven Promotors, wie z. B. des GAPDH-Promotors. Werden die beiden Expressionskonstrukte auf verschiedenen Plasmiden bzw. auf einem Plasmid in verschiedenen Cistronen exprimiert, so können dafür gleiche oder jeweils ein unterschiedlicher Promotor benutzt werden. Bevorzugt werden unterschiedliche Promotoren.

Die Expressionskonstrukte (Promotor-Signalsequenzrekombinantes-Gen) für den zu produzierenden Volllängen-Antikörper werden danach unter Verwendung von dem Fachmann bekannten Methoden in die Zellen von E. coli-Stämmen, die periplasmatische Proteine ins Medium abgeben, eingebracht.

Dies erfolgt z. B. auf einem Vektor, z. B. einem Plasmid, wie etwa einem Abkömmling von bekannten Expressionsvektoren, wie pJF118EH, pKK223-3, pUC18, pBR322, pACYC184, pACYC177, pASK-IBA3 oder pET. Als Selektionsmarker für Plasmide geeignet sind Gene, die für eine Resistenz gegenüber z. B. Ampicillin, Tetracyclin, Chloramphenicol, Kanamycin oder andere Antibiotika codieren. Besonders bevorzugte Antibiotika sind Tetracyclin und Kanamycin. Werden zwei kompatible Plasmide verwendet, werden üblicherweise unterschiedliche Selektionsmarker benutzt.

Die Erfindung betrifft somit auch einen E. coli Stamm, welcher ausgewählt ist aus der Gruppe BLR, K802, WCM100, MM28, RV308, RR1, "Leaky"-Mutante von E. coli-Stämmen und welcher periplasmatische Proteine ins Medium abgibt, enthaltend ein Gen kodierend für eine schwere Kette eines Antikörpers funktionell verknüpft mit einer Signalsequenz kodierend für ein Signalpeptid sowie ein zweites Gen kodierend für eine leichte Kette eines Antikörpers, funktionell verknüpft mit einer Signalsequenz kodierend für ein Signalpeptid.

Im erfindungsgemäßen E. coli Stamm ist das Gen kodierend für eine schwere Kette eines Antikörpers funktionell verknüpft mit einer Signalsequenz kodierend für ein Signalpeptid sowie das zweite Gen kodierend für eine leichte Kette eines Antikörpers, funktionell verknüpft mit einer Signalsequenz kodierend für ein Signalpeptid, vorzugsweise ferner mit in E. coli funktionellen Expressionssignalen, vorzugsweise einem Promotor, einem Transkriptions-, Translationsstart, einer Ribosomenbindestelle, und einem Terminator versehen. Es handelt sich dabei vorzugsweise um die bereits vorstehend genannten Expressionssignale.

Die Kultivierung der mit dem Expressionsplasmid transformierten Zellen erfolgt nach an und für sich üblichen, dem Fachmann bekannten Fermentationsverfahren in Bioreaktoren (Fermentern).

Die Fermentation findet vorzugsweise in einem üblichen Bioreaktor, beispielswiese einem Rührkessel, einem Blasensäulen-Fermenter oder einem Airlift-Fermenter statt. Besonders bevorzugt ist ein Rührkessel-Fermenter. Dabei werden die Zellen des Protein-Produktionsstammes in einem Flüssigmedium über einen Zeitraum von 16 - 150 h kultiviert, wobei verschiedene Parameter, wie z. B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24 - 72 h.

Als Fermentationsmedien kommen alle gängigen, dem Fachmann bekannten Medien für die Kultivierung von Mikroorganismen in Frage. Es können sowohl Komplex- oder Vollmedien, oder Minimalsalzmedien, die im Gegensatz zum Vollmedium eine genau definierte Substrat-Zusammensetzung aufweisen oder Minimalsalzmedien, denen ein bestimmter Anteil von Komplex-Komponenten wie z. B. Pepton, Trypton, Hefeextrakt, Melasse oder Corn Steep liquor zugesetzt wird, benützt werden.

Als primäre Kohlenstoffquelle können prinzipiell alle von den Zellen verwertbaren Zucker, Zuckeralkohole oder organische Säuren bzw. deren Salze verwendet werden. Dabei werden bevorzugt Glucose, Laktose oder Glycerin eingesetzt. Besonders bevorzugt sind Glucose und Laktose. Auch ist eine kombinierte Fütterung mehrerer verschiedener Kohlenstoffquellen möglich. Die Kohlenstoffquelle wird im Medium vorgelegt,
z. B. in einer Konzentration von 10 - 30 g/l, und wird dann je nach Bedarf der Kultur von außen zugefüttert.

Der Sauerstoff-Partialdruck (pO₂) in der Kultur beträgt vorzugsweise zwischen 10 und 70 % Sättigung. Bevorzugt wird ein pO₂ zwischen 30 und 60 %, besonders bevorzugt liegt der pO₂ zwischen 45 und 55 % Sättigung.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45°C. Bevorzugt ist ein Temperaturbereich zwischen 20 und 40°C, besonders bevorzugt ist ein Temperaturbereich zwischen 25 und 35°C, ganz besonders bevorzugt sind 30°C.

In einer bevorzugten Ausführungsform wird die Temperatur im Verlauf der Fermentation nicht konstant gehalten, sondern beispielsweise vor Induktion der Genexpression abgesenkt, um so eine mögliche Bildung von "Inclusion bodies" zu verhindern. In so einem Fall ist die Absenkung von 30°C auf 25°C bevorzugt. Besonders bevorzugt ist eine Absenkung von 30 auf 20°C.

Die Aufreinigung des ausgeschiedenen Volllängen-Antikörpers kann durch bekannte Aufreinigungsmethoden geschehen. Üblicherweise werden in einem ersten Schritt durch Separationsmethoden, wie Zentrifugation oder Filtration, die Zellen von dem sekretierten Antikörper abgetrennt. Der Antikörper kann z. B. durch Ultrafiltration aufkonzentriert werden und wird dann über Standardmethoden, wie Fällung, Chromatographie oder Ultrafiltration weiter gereinigt. Besonders bevorzugt sind hierbei

Methoden, wie Affinitätschromatographie, bei der die bereits korrekt gefaltete native Konformation des Antikörpers ausgenützt wird.
Fig. 1 zeigt den Klonierungsvektor pJF118ut aus dem Beispiel.
Fig. 2 zeigt das Plasmid pHC-Anti-TF aus dem Beispiel.
Fig. 3 zeigt das Anti-TF-Antikörper Expressionsplasmid pAK-Anti-TF aus dem Beispiel.

Die in den Figuren verwendeten Abkürzungen haben die folgende Bedeutung:
tac p/o: tac-Promotor/Operator
rrnB: Terminator
bla:β-Lactamase-Gen (Ampicillin-Resistenz)
ColE1: Origin of Replication
TcR: Tetracyclin-Resistenz
lacIq: Repressor des tac-Promotors
SD: Shine-Dalgarno-Sequenz
ompA-SP: ompA-Signalpeptid
HC (Anti-TF): Leserahmen der schweren Kette des Anti-TF-Antikörpers
cgt-SP: Signalpeptid der CGTase
LC (Anti-TF): Leserahmen der leichten Kette des Anti-TF-Antikörpers

Das folgende Beispiel dient der weiteren Erläuterung der Erfindung.

Sämtliche eingesetzten molekularbiologischen Verfahren, wie Polymerase-Ketten-Reaktion, Gensynthese, Isolierung und Reinigung von DNS, Modifikation von DNS durch Restriktionsenzyme, Klenow-Fragment und Ligase, Transformation etc., wurden in der dem Fachmann bekannten, in der Literatur beschriebenen oder von den jeweiligen Herstellern empfohlenen Art und Weise durchgeführt.

### Beispiel: Fermentative Produktion von Volllängen-Antikörpern mit Hilfe von E. coli Sekretionsmutanten im 10 1-Maßstab

Das vorliegende Beispiel beschreibt die Produktion des Anti-Tissue factor (αTF) IgG1-Antikörpers.

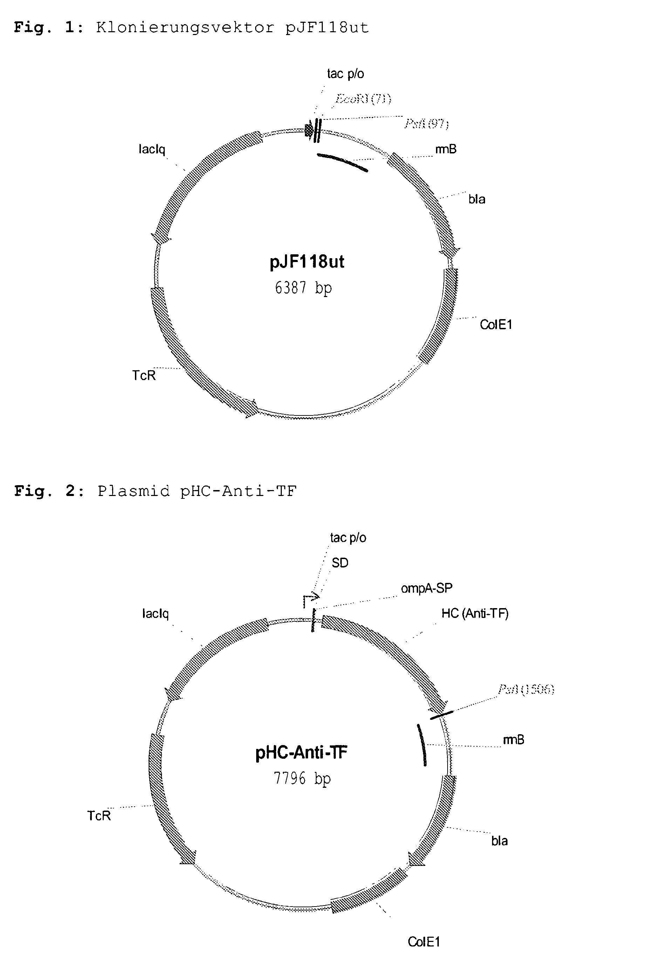

Als Ausgangsvektor für die Klonierung und Expression der Gene des Anti-αTF-Antikörpers diente das Plasmid pJF118ut (Fig.1), welches bei der DSMZ-Deutsche Sammlung von Mikroorganismen und Zellkulturen GmbH (Braunschweig) unter der Nummer DSM 18596 hinterlegt ist. pJF118ut ist ein Derivat des bekannten Expressionsvektors pKK223-3 (Amersham Pharmacia Biotech) und enthält neben dem β-Lactamase-Gen und dem Tetracyclin-Resistenzgen noch den tac-Promotor, der durch das LacIq-Genprodukt, dessen Gen ebenfalls auf dem Plasmid vorliegt, reprimiert wird und der durch einen Induktor, wie z. B. D-Lactose oder Isopropyl-β-D-thiogalactopyranosid (IPTG), angeschaltet werden kann.

In dieses Plasmid wurden in zwei aufeinanderfolgenden Schritten die beiden Leserahmen für die schwere Kette bzw. für die leichte Kette des Anti-αTF-Antikörpers jeweils inklusive einer Signalsequenz kloniert.

Dazu wurde wie folgt vorgegangen:

Das DNS-Fragment mit der SEQ ID No. 2 (schwere Kette) wurde mittels Gensynthese hergestellt und umfasst eine Genfusion bestehend aus der Signalsequenz des ompA-Gens von *E. coli* und dem Leserahmen für die schwere Kette des Anti-αTF-Antikörpers. Dieses DNS-Fragment wurde zunächst mit den Restriktionsenzymen EcoRI und PstI geschnitten und mit dem Expressionsvektor pJF118ut, der mit denselben Restriktionsenzymen geschnitten worden war, ligiert. Das aus dieser Klonierung resultierende Plasmid, bei dem die Expression des Gens für die schwere Kette unter der Kontrolle des tac-Promotors liegt, wurde mit pHC-Anti-TF bezeichnet (Fig. 2)

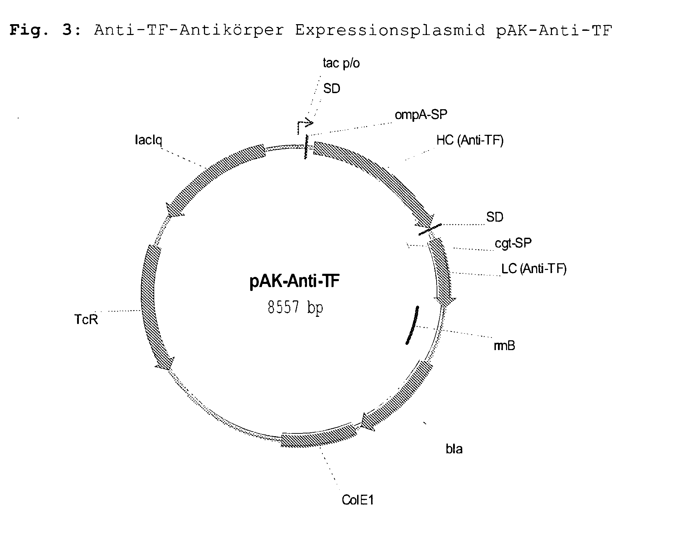

Das DNS-Fragment mit der SEQ ID No. 3 (leichte Kette) wurde ebenfalls mittels Gensynthese hergestellt und umfasst eine Genfusion bestehend aus der Signalsequenz einer CGTase und dem Leserahmen für die leichte Kette des Anti-αTF-Antikörpers. Dieses DNS-Fragment wurde zunächst mit dem Restriktionsenzym PstI geschnitten und anschließend mit dem Vektor pHC-Anti-TF, der mit demselben Restriktionsenzym geschnittenen worden war, ligiert. Das daraus resultierende Plasmid wurde mit pAK-Anti-TF (Fig. 3) bezeichnet. Auf diese Weise wurde ein artifizielles Operon bestehend aus den jeweiligen Leserahmen für die schwere und die leichte Kette erzeugt, welches unter der Kontrolle des tac-Promotors steht. Damit ist durch Zugabe eines Induktors (z. B. IPTG) eine synchrone Expression der beiden Gene möglich.

Für die Herstellung des Anti-αTF-Antikörpers wurden die Stämme jeweils mit dem Plasmid pAK-Anti-TF mit der CaCl₂-Methode transformiert. Die Selektion auf plasmidhaltige Zellen erfolgte mittels Ampicillin (100 mg/l).

Die Produktion wurde in 10 l Rührkesselfermentern durchgeführt.

Der mit 6 l des Mediums FM4 (1,5 g/l KH₂PO₄; 5 g/l (NH₄)₂SO₄; 0,3 g/l MgSO₄ x 7 H₂O; 0,075 g/l FeSO₄ x 7 H₂O; 1 g/l Na₃Citrat x 2 H₂O; 0,5 g/l NaCl; 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B₁; 3 g/l Phyton; 1,5 g/l Hefeextrakt; 10 g/l Glukose; 100 mg/l Ampicillin) befüllte Fermenter wurde im Verhältnis 1:10 mit einer Vorkultur, die übernacht im gleichen Medium kultiviert wurde, angeimpft. Während der Fermentation wurde eine Temperatur von 30°C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine maximale Glukose-Konzentration im Medium von < 10 g/l angestrebt wurde. Die Induktion der Expression erfolgte durch Zugabe von Isopropyl-β-D-thiogalactopyranosid (IPTG) ad 0,1 mM am Ende der logarithmischen Wachstumsphase. Nach 72 h Fermentation wurden Proben entnommen und anschließend die Zellen durch Zentrifugation vom Kulturmedium abgetrennt.

Die Quantifizierung des in das Kulturmedium sekretierten Anti-αTF-Antikörpers erfolgte über eine Aktivitäts-Bestimmung mit einem ELISA-Test mit löslichem Tissue factor als Antigen (Coating) und einem Peroxidase-konjugierten goat Anti-human F(ab')₂-Fragment als sekundärem Antikörper, wie in Simmons et al. (2002, J. Immunol. Methods 263, 133 - 47) beschrieben.

In Tabelle 1 sind die auf diese Weise bestimmten Ausbeuten an funktionellem Anti-αTF-Antikörper aufgelistet.

**Tabelle 1: Anti-αTF-Antikörper-Ausbeuten im Kulturüberstand nach 72 h Fermentation**

| Stamm | Anti-OGTF-Antikörper [mg/l] |
|---|---|
| BLR/pAK-Anti-TF | 160 |
| WCM100/pAK-Anti-TF | 240 |
| JF733/pAK-Anti-TF | 170 |
| A592/pAK-Anti-TF | 180 |
| JE5505/pAK-Anti-TF | 160 |
| AE84064//pAK-Anti-TF | 200 |

## Patentansprüche

1. Verfahren zur Herstellung eines korrekt gefalteten und assemblierten Volllängen-Antikörpers mittels eines *E*. *coli-*Stammes, **dadurch gekennzeichnet, dass** ein *E*. *coli-*Stamm, welcher ausgewählt ist aus der Gruppe BLR, K802, WCM100, MM28, RV308, RR1, "Leaky"-Mutante von E. coli-Stämmen und welcher periplasmatische Proteine ins Medium abgibt, enthaltend ein Gen kodierend für die schwere Kette eines Antikörpers funktionell verknüpft mit einer Signalsequenz kodierend für ein Signalpeptid sowie ein zweites Gen kodierend für die leichte Kette eines Antikörpers, funktionell verknüpft mit einer Signalsequenz kodierend für ein Signalpeptid, in einem Kulturmedium fermentiert wird, wobei der *E. coli*-Stamm einen Volllängen-Antikörper in das Kulturmedium ausscheidet und der Volllängen Antikörper vom Kulturmedium abgetrennt wird.

2. Verfahren gemäß Anspruch 1, **dadurch gekennzeichnet, dass** die "Leaky"-Mutante eine Mutation in einem der folgenden Gene aufweist: omp-Gene, tol-Gene, excD-Gen, excC-Gen, lpp-Gen, env-Genen und lky-Gene.

3. Verfahren gemäß Anspruch 1 oder 2, **dadurch gekennzeichnet, dass** es sich bei der "Leaky"-Mutante um einen E. coli-Stamm aus der folgenden Gruppe handelt: JF733, A592, A593, A586, CAG12184, G11e1, JE5511, E610, E623, JE5505, PM61, 6628, PM61R, JP1228, 207, AE84064.

4. Verfahren gemäß einem der Ansprüche 1 bis 3, **dadurch gekennzeichnet, dass** der Antikörper in einer Ausbeute von > 160 mg/l ohne Koexpression eines periplasmatischen Chaperons erhalten wird.

5. Verfahren gemäß einem der Ansprüche 1 bis 4, **dadurch gekennzeichnet, dass** die Signalsequenz kodierend für ein Signalpeptid ausgewählt ist aus den Signalsequenzen des *phoA-* oder ompA-Gens von *E. coli* oder der Signalsequenz mit der Sequenz SEQ ID NO: 1.

6. Verfahren gemäß einem der Ansprüche 1 bis 5, **dadurch gekennzeichnet, dass** die 5'-Enden der Gene der leichten und schweren Kette in frame mit unterschiedlichen Signalsequenzen kodierend für ein Signalpeptid verknüpft sind.

7. Verfahren gemäß Anspruch 6, **dadurch gekennzeichnet, dass** das 5'-Ende des Gens kodierend für die eine Kette des Antikörpers in frame mit der Signalsequenz des *phoA-* oder *ompA*-Gens von *E. coli* funktionell verknüpft ist und das 5'-Ende des Gens kodierend für die anderen Kette des Antikörpers in frame mit der Signalsequenz SEQ ID NO: 1 verknüpft ist.

8. Verfahren gemäß einem der Ansprüche 1 bis 7, **dadurch gekennzeichnet, dass** das Gen kodierend für die schwere Kette eines Antikörpers funktionell verknüpft mit einer Signalsequenz kodierend für ein Signalpeptid sowie das zweite Gen kodierend für die leichte Kette eines Antikörpers, funktionell verknüpft mit einer Signalsequenz kodierend für ein Signalpeptid in einem Operon zusammengefasst sind.

9. Verfahren gemäß einem der Ansprüche 1 bis 8, **dadurch gekennzeichnet, dass** über einen Zeitraum von 16 - 150 h fermentiert wird.

10. Verfahren gemäß einem der Ansprüche 1 bis 9, **dadurch gekennzeichnet, dass** die Fermentation bei einem Sauerstoff-Partialdruck (pO₂) zwischen 10 und 70 % Sättigung, bevorzugt zwischen 30 und 60 %, besonders bevorzugt zwischen 45 und 55 % Sättigung erfolgt.

11. Verfahren gemäß einem der Ansprüche 1 bis 10, **dadurch gekennzeichnet, dass** der pH-Wert im Kulturmedium zwischen pH 6 und pH 8, bevorzugt zwischen pH 6,5 und 7,5, besonders bevorzugt zwischen 6,8 und 7,2 liegt.

12. Verfahren gemäß einem der Ansprüche 1 bis 11, **dadurch gekennzeichnet, dass** die Temperatur vor Induktion der Genexpression von 30°C auf 25°C, bevorzugt von 30 auf 20°C abgesenkt wird und so eine Bildung von "Inclusion bodies" verhindert wird.

13. **Verfahren gemäß einem der Ansprüche 1 bis 12, dadurch gekennzeichnet, dass** die Abtrennung der Volllängen Antikörper vom Kulturmedium in einem ersten Schritt durch Separationsmethoden, wie Zentrifugation oder Filtration, erfolgt, der Antikörper dann durch Ultrafiltration aufkonzentriert wird und anschließend mittels Fällung, Chromatographie oder Ultrafiltration, besonders bevorzugt mittels Affinitätschromatographie weiter gereinigt wird.

14. E. coli Stamm zur Herstellung eines korrekt gefalteten Volllängen-Antikörpers ausgewählt aus der Gruppe BLR, K802, WCM100, MM28, RV308, RR1, "Leaky"-Mutante von E. coli-Stämmen und welcher periplasmatische Proteine ins Medium abgibt, enthaltend ein Gen kodierend für eine schwere Kette eines Antikörpers funktionell verknüpft mit einer Signalsequenz kodierend für ein Signalpeptid sowie ein zweites Gen kodierend für eine leichte Kette eines Antikörpers, funktionell verknüpft mit einer Signalsequenz kodierend für ein Signalpeptid.

15. E. coli Stamm gemäß Anspruch 14, **dadurch gekennzeichnet, dass** das Gen kodierend für eine schwere Kette eines Antikörpers funktionell verknüpft mit einer Signalsequenz kodierend für ein Signalpeptid sowie das zweite Gen kodierend für eine leichte Kette eines Antikörpers, funktionell verknüpft mit einer Signalsequenz kodierend für ein Signalpeptid mit in E. coli funktionellen Expressionssignalen, vorzugsweise einem Promotor, einem Transkriptions-, Translationsstart, einer Ribosomenbindestelle, und einem Terminator versehen sind.

## Claims

1. Process for producing a correctly folded and assembled full-length antibody using an *E. coli* strain, **characterized in that** an *E. coli* strain which is selected from the group of BLR, K802, WCM100, MM28, RV308, RR1, leaky mutants of E. coli strains and which releases periplasmic proteins into the medium, comprising a gene coding for the heavy chain of an antibody functionally linked to a signal sequence coding for a signal peptide, and a second gene coding for the light chain of an antibody, functionally linked to a signal sequence coding for a signal peptide, is fermented in a culture medium, where the *E. coli* strain secretes a full-length antibody into the culture medium, and the full-length antibody is removed from the culture medium.

2. Process according to Claim 1, **characterized in that** the leaky mutant has a mutation in one of the following genes: omp genes, tol genes, excD gene, excC gene, lpp gene, env genes and lky genes.

3. Process according to Claim 1 or 2, **characterized in that** the leaky mutant is an E. coli strain from the following group: JF733, A592, A593, A586, CAG12184, G11e1, JE5511, E610, E623, JE5505, PM61, 6628, PM61R, JP1228, 207, AE84064.

4. Process according to any of Claims 1 to 3, **characterized in that** the antibody is obtained in a yield of > 160 mg/l without coexpression of a periplasmic chaperone.

5. Process according to any of Claims 1 to 4, **characterized in that** the signal sequence coding for a signal peptide is selected from the signal sequences of the *pho*A or *omp*A gene of *E. coli* or the signal sequence having sequence SEQ ID NO: 1.

6. Process according to any of Claims 1 to 5, **characterized in that** the 5' ends of the genes of the light and heavy chain are linked in frame to different signal sequences coding for a signal peptide.

7. Process according to Claim 6, **characterized in that** the 5' end of the gene coding for the one chain of the antibody is functionally linked in frame to the signal sequence of the *pho*A or *omp*A gene of *E. coli*, and the 5' end of the gene coding for the other chain of the antibody is linked in frame to the signal sequence SEQ ID NO: 1.

8. Process according to any of Claims 1 to 7, **characterized in that** the gene coding for the heavy chain of an antibody functionally linked to a signal sequence coding for a signal peptide, and the second gene coding for the light chain of an antibody, functionally linked to a signal sequence coding for a signal peptide, are combined in one operon.

9. Process according to any of Claims 1 to 8, **characterized in that** fermentation takes place over a period of 16 - 150 h.

10. Process according to any of Claims 1 to 9, **characterized in that** the fermentation takes place with an oxygen partial pressure (pO₂) of between 10 and 70% saturation, preferably between 30 and 60%, particularly preferably between 45 and 55% saturation.

11. Process according to any of Claims 1 to 10, **characterized in that** the pH in the culture medium is between pH 6 and pH 8, preferably between pH 6.5 and 7.5, particularly preferably between 6.8 and 7.2.

12. Process according to any of Claims 1 to 11, **characterized in that** the temperature before induction of gene expression is reduced from 30°C to 25°C, preferably from 30 to 20°C, and thus formation of inclusion bodies is prevented.

13. Process according to any of Claims 1 to 12, **characterized in that** the removal of the full-length antibodies from the culture medium takes place in a first step by separation methods such as centrifugation or filtration, the antibody is then concentrated by ultrafiltration and is subsequently further purified by precipitation, chromatography or ultrafiltration, particularly preferably by affinity chromatography.

14. E. coli strain for producing a correctly folded full-length antibody, selected from the group of BLR, K802, WCM100, MM28, RV308, RR1, leaky mutants of E. coli strains and which releases periplasmic proteins into the medium, comprising a gene coding for a heavy chain of an antibody functionally linked to a signal sequence coding for a signal peptide, and a second gene coding for a light chain of an antibody, functionally linked to a signal sequence coding for a signal peptide.

15. E. coli strain according to Claim 14, **characterized in that** the gene coding for a heavy chain of an antibody functionally linked to a signal sequence coding for a signal peptide, and the second gene coding for a light chain of an antibody, functionally linked to a signal sequence coding for a signal peptide, are provided with expression signals which are functional in E. coli, preferably a promoter, a transcription start, translation start, a ribosome binding site, and a terminator.

## Revendications

1. Procédé de fabrication d'un anticorps de pleine longueur correctement plié et assemblé au moyen d'une souche d'*E. coli*, **caractérisé en ce qu'**une souche d'*E. coli*, qui est choisie dans le groupe comprenant BLR, K802, WCM100, MM28, RV308, RR1, le mutant « Leaky » de souches d'E. coli et qui libère des protéines périplasmiques dans le milieu, contenant un gène qui code pour la chaîne lourde d'un anticorps, relié fonctionnellement avec une séquence signal qui code pour un peptide signal, ainsi qu'un second gène qui code pour la chaîne légère d'un anticorps, relié fonctionnellement avec une séquence signal qui code pour un peptide signal, est fermentée dans un milieu de culture, la souche d'*E. coli* excrétant un anticorps de pleine longueur dans le milieu de culture et l'anticorps de pleine longueur étant séparé du milieu de culture.

2. Procédé selon la revendication 1, **caractérisé en ce que** le mutant « Leaky » présente une mutation dans un des gènes suivants : les gènes omp, les gènes tol, le gène excD, le gène excC, le gène lpp, les gènes env et les gènes lky.

3. Procédé selon la revendication 1 ou 2, **caractérisé en ce que** le mutant « Leaky » est une souche d'E. coli du groupe suivant : JF733, A592, A593, A586, CAG12184, G11e1, JE5511, E610, E623, JE5505, PM61, 6628, PM61R, JP1228, 207, AE84064.

4. Procédé selon l'une quelconque des revendications 1 à 3, **caractérisé en ce que** l'anticorps est obtenu en un rendement > 160 mg/l sans coexpression d'un chaperon périplasmique.

5. Procédé selon l'une quelconque des revendications 1 à 4, **caractérisé en ce que** la séquence signal codant pour un peptide signal est choisie parmi les séquences signal du gène *pho*A ou *omp*A d'*E. coli* ou la séquence signal de séquence SEQ ID NO : 1.

6. Procédé selon l'une quelconque des revendications 1 à 5, **caractérisé en ce que** les extrémités 5' des gènes de la chaîne légère et de la chaîne lourde sont reliées en phase avec différentes séquences signal codant pour un peptide signal.

7. Procédé selon la revendication 6, **caractérisé en ce que** l'extrémité 5' du gène codant pour une chaîne de l'anticorps est reliée fonctionnellement en phase avec la séquence signal du gène *pho*A ou *omp*A d'*E. coli* et l'extrémité 5' du gène codant pour l'autre chaîne de l'anticorps est reliée en phase avec la séquence signal SEQ ID NO : 1.

8. Procédé selon l'une quelconque des revendications 1 à 7, **caractérisé en ce que** le gène codant pour la chaîne lourde d'un anticorps, relié fonctionnellement avec une séquence signal codant pour un peptide signal, et le second gène codant pour la chaîne légère d'un anticorps, relié fonctionnellement avec une séquence signal codant pour un peptide signal, sont rassemblés dans un opéron.

9. Procédé selon l'une quelconque des revendications 1 à 8, **caractérisé en ce que** la fermentation est réalisée pendant une durée de 16 à 150 h.

10. Procédé selon l'une quelconque des revendications 1 à 9, **caractérisé en ce que** la fermentation a lieu à une pression partielle en oxygène (pO₂) comprise entre 10 et 70 % de saturation, de préférence entre 30 et 60 %, de manière particulièrement préférée entre 45 et 55 % de saturation.

11. Procédé selon l'une quelconque des revendications 1 à 10, **caractérisé en ce que** le pH dans le milieu de culture est compris entre 6 et 8, de préférence entre 6,5 et 7,5, de manière particulièrement préférée entre 6,8 et 7,2.

12. Procédé selon l'une quelconque des revendications 1 à 11, **caractérisé en ce que** la température est abaissée avant l'induction de l'expression des gènes de 30 °C à 25 °C, de préférence de 30 à 20 °C, et une formation de corps d'inclusion est ainsi empêchée.

13. Procédé selon l'une quelconque des revendications 1 à 12, **caractérisé en ce que** la séparation des anticorps de pleine longueur du milieu de culture a lieu lors d'une première étape par des procédés de séparation, tels que la centrifugation ou la filtration, puis l'anticorps est concentré par ultrafiltration, et ensuite davantage purifié par précipitation, chromatographie ou ultrafiltration, de manière particulièrement préférée par chromatographie d'affinité.

14. Souche d'E. coli pour la fabrication d'un anticorps de pleine longueur correctement plié, choisie dans le groupe comprenant BLR, K802, WCM100, MM28, RV308, RR1, les mutants « Leaky » de souches d'E. coli et qui libère des protéines périplasmiques dans le milieu, contenant un gène qui code pour une chaîne lourde d'un anticorps, relié fonctionnellement avec une séquence signal qui code pour un peptide signal, ainsi qu'un second gène qui code pour une chaîne légère d'un anticorps, relié fonctionnellement avec une séquence signal qui code pour un peptide signal.

15. Souche d'E. coli selon la revendication 14, **caractérisée en ce que** le gène codant pour une chaîne lourde d'un anticorps, relié fonctionnellement avec une séquence signal codant pour un peptide signal, et le second gène codant pour une chaîne légère d'un anticorps, relié fonctionnellement avec une séquence signal codant pour un peptide signal, sont munis de signaux d'expression fonctionnels dans E. coli, de préférence d'un promoteur, d'un démarreur de transcription, d'un démarreur de traduction, d'un emplacement de liaison au ribosome et d'un terminateur.


### SEQUENZPROTOKOLL

<110> Consortium fuer elektrochemische Industrie GmbH
<120> Verfahren zur fermentativen Herstellung von Antikoerpern
<130> Co10616
<140>
   <141>
<160> 3
<170> PatentIn Vers. 2.0
<210> 1
   <211> 90
   <212> DNA
   <213> Klebsiella pneumoniae
<220>
   <223> CGTase-Signalsequenz
<400> 1
   atgaaaagaa accgtttttt taatacctcg gctgctattg ccatttcgat tgcattaaat 60
   actttttttt gtagcatgca gacgattgct                                  90
<210> 2
   <211> 1441
   <212> DNA
   <213> Artificial Sequence
<220>
   <223> DNS-Molekuel, welches das Gen fuer die schwere Kette des Anti-TF-Antikoerpers enthaelt
<220>
   <221> sig_peptide
   <222> (24)..(86)
   <223> ompA-Signalsequenz
<220>
   <221> allele
   <222> (87)..(1430)
   <223> Gen der schweren Kette des Anti-TF-Antikoerpers
<400> 2
   cagaattcta aggaggaaat tatatgaaaa agacagctat cgcgattgca gtggcactgg 60
   ctggtttcgc taccgtagcg caggctgagg ttcagctggt ggagtctggc ggtggcctgg 120
   tgcagccagg gggctcactc cgtttgtcct gtgcagcttc tggcttcaat attaaggagt 180
   actacatgca ctgggtccgt caggccccgg gtaagggcct ggaatgggtt ggattgattg 240
   atccagagca aggcaacacg atctatgacc cgaagttcca ggaccgtgcc actataagcg 300
   ctgacaattc caaaaacaca gcatacctgc aaatgaacag cctgcgtgct gaggacactg 360
   ccgtctatta ttgtgctcga gacacggccg cttacttcga ctactggggt caaggaaccc 420
   tggtcaccgt ctcctcggcc tccaccaagg gcccatcggt cttccccctg gcaccctcct 480
   ccaagagcac ctctgggggc acagcggccc tgggctgcct ggtcaaggac tacttccccg 540
   aaccggtgac ggtgtcgtgg aactcaggcg ccctgaccag cggcgtgcac accttcccgg 600
   ctgtcctaca gtcctcagga ctctactccc tcagcagcgt ggtgactgtg ccctctagca 660
   gcttgggcac ccagacctac atctgcaacg tgaatcacaa gcccagcaac accaaggtgg 720
   acaagaaagt tgagcccaaa tcttgtgaca aaactcacac atgcccaccg tgcccagcac 780
   ctgaactcct ggggggaccg tcagtcttcc tcttcccccc aaaacccaag gacaccctca 840
   tgatctcccg gacccctgag gtcacatgcg tggtggtgga cgtgagccac gaagaccctg 900
   aggtcaagtt caactggtac gtggacggcg tggaggtgca taatgccaag acaaagccgc 960
   gggaggagca gtacaacagc acgtaccgtg tggtcagcgt cctcaccgtc ctgcaccagg 1020
   actggctgaa tggcaaggag tacaagtgca aggtctccaa caaagccctc ccagccccca 1080
   tcgagaaaac catctccaaa gccaaagggc agccccgaga accacaggtg tacaccctgc 1140
   ccccatcccg ggaagagatg accaagaacc aggtcagcct gacctgcctg gtcaaaggct 1200
   tctatcccag cgacatcgcc gtggagtggg agagcaatgg gcagccggag aacaactaca 1260
   agaccacgcc tcccgtgctg gactccgacg gctccttctt cctctacagc aagctcaccg 1320
   tggacaagag caggtggcag caggggaacg tcttctcatg ctccgtgatg catgaggctc 1380
   tgcacaacca ctacacgcag aagagcctct ccctgtctcc gggtaaataa taactgcaga 1440
   a 1441
<210> 3
   <211> 771
   <212> DNA
   <213> Artificial Sequence
<220>
   <223> DNS-Molekuel, welches das Gen fuer die leichte Kette des Anti-TF-Antikoerpers enthaelt
<220>
   <221> sig_peptide
   <222> (26)..(115)
   <223> CGTase-Signalsequenz
<220>
   <221> allele
   <222> (116) .. (760)
   <223> Gen der leichten Kette des Anti-TF-Antikoerpers
<400> 3
   aactgcagta catggagaaa ataaaatgaa aagaaaccgt ttttttaata cctcggctgc 60
   tattgccatt tcgattgcat taaatacttt tttttgtagc atgcagacga ttgctgatat 120
   ccagatgacc cagtccccga gctccctgtc cgcctctgtg ggcgataggg tcaccatcac 180
   gtgcagagcc agtcgcgaca tcaagagcta tctgaactgg tatcaacaga aaccaggaaa 240
   agctccgaaa gtactgattt actatgctac tagtctcgct gaaggagtcc cttctcgctt 300
   ctctggatcc ggttctggga cggattacac tctgaccatc agcagtctgc aaccagaaga 360
   cttcgcaact tattactgtc ttcagcacgg agagtctccg tggacatttg gacagggtac 420
   caaggtggag atcaaacgaa ctgtggctgc accatctgtc ttcatcttcc cgccatctga 480
   tgagcagttg aaatctggaa ctgcttctgt tgtgtgcctg ctgaataact tctatcccag 540
   agaggccaaa gtacagtgga aggtggataa cgccctccaa tcgggtaact cccaggagag 600
   tgtcacagag caggacagca aggacagcac ctacagcctc agcagcaccc tgacgctgag 660
   caaagcagac tacgagaaac acaaagtcta cgcctgcgaa gtcacccatc agggcctgag 720
   ctcgcccgtc acaaagagct tcaacagggg agagtgttaa tagctgcaga a          771